# EUROPÄISCHE PATENTANMELDUNG

(11) **EP 0 891 779 A1**
(43) Veröffentlichungstag der Anmeldung: **20.01.1999**
(21) Anmeldenummer: 97110026.8
(22) Anmeldetag: 19.06.1997
(51) Int. Cl.: A61K 38/29

(54) **Pharmazeutische Kombinationspräparate enthaltend Parathyroidhormon und Calcium- und/oder Phosphatverbindungen**

(71) Anmelder: BOEHRINGER MANNHEIM GMBH, 68298 Mannheim (DE)
(72) Erfinder: Lehmann, Paul, Dr.,Dipl.-Chem., 67549 Worms (DE)

(57) **Zusammenfassung**

Die vorliegende Erfindung betrifft pharmazeutische Kombinationspräparate enthaltend Parathyroidhormon (PTH) und Calcium- und/oder Phosphatverbindungen. Diese Kombinationspräparate werden insbesondere zur Behandlung von Knochenstoffwechselerkrankungen eingesetzt.

## Beschreibung

Die vorliegende Erfindung betrifft pharmazeutische Kombinationspräparate enthaltend Parathyroidhormon (PTH) und Calcium- und/oder Phosphatverbindungen. Diese Kombinationspräparate werden insbesondere zur Behandlung von Knochenstoffwechselerkrankungen eingesetzt.

Das Parathyroidhormon (PTH), ein Hormon (84 Aminosäuren) der Nebenschilddrüsen, ist ein wichtiger Regulator zur Aufrechterhaltung des Calciumspiegels im Körper. PTH kann die Knochenbildung oder Knochenresorption stimulieren. Es wirkt dabei als regulatorisches Hormon auf eine Reihe von Enzymen, unter anderem auf die Ornithindecarboxylase und Adenylatcyclase (cAMP-Synthese). PTH mobilisiert bei Calcium-Mangel Calcium aus dem Knochen, verringert die Calciumausscheidung der Nieren und verbessert gleichzeitig die Resorption von Calcium aus dem Darm durch erhöhte Synthese von 1,25 Dihydroxycholcalciferol. Durch die Wirkung auf diese Zielorgane wird eine Normalisierung des Calciumspiegels erreicht. Umgekehrt wird bei erhöhtem Calciumspiegel der Einbau von Calcium in den Knochen stimuliert. Ferner zeigt PTH einen mitogenen Effekt, insbesondere eine Stimulation von Osteoblasten und Chondrocyten.

Diese knochenanabole Wirkung des PTH wurde in tierexperimentellen Untersuchungen an Ratten sowie in klinischen Untersuchungen an osteoporotischen Patienten beobachtet und in der Fachliteratur beschrieben (Selye, Endocrinology 16 (1932), 547-558; Hefti et al., Clin. Sci. 62(1982), 389-396; Gunnes-Hey et al., Metab. Bone Dis. Relat. Res. 5 (1984), 177 - 181; Reeve et al., Br. Med. J. 280 (1980), 1340-1344; Slovik et a1., J. Bone Miner. Res. 1(1986), 377-381; EP 0197514).

Das Knochengewebe ist das Speicherorgan für Calciumionen aus dem sie bei Mangelzuständen mobilisiert werden können. Erkrankungen des Knochengewebes sind häufig Prozesse, die überwiegend im Rahmen von Krankheiten anderer Organe oder des gesamten Stoffwechsels auftreten. Sie können durch die Störung der hormonellen Regulation der Mineralisation und des Knochenaufbaus und Knochenabbaus zu Demineralisationserscheinungen (Osteomolazie), Knochenabbau (Osteoporose) oder Knochendeformation ehren.
Knochengewebe besteht histologisch aus 5-10 µm dicken Lamellen der Knochensubstanz und darin eingelagerten Zellen, den Osteozyten, die um die entlang der Längsachse laufenden kleinen Gefäße des Knochens angeordnet sind. Die an der Oberfläche des Knochens gelegenen Osteoblasten sind am Aufbau der Knochensubstanz beteiligt. Ihre Tätigkeit steht in einem hormonell gesteuerten Gleichgewicht mit dem Knochenabbau durch die Osteoklasten.

Das für die Festigkeit des Knochens notwendige Knochenmineral, das 70% der Knochensubstanz ausmacht, besteht aus Calcium und Phosphat. Der organische Anteil, der 30% der Knochensubstanz ausmacht, setzt sich überwiegend aus einer ProteoglykanMatrix und einem Fasernetz aus Kollagen Typ I zusammen.

Im nativen Knochen besteht die mineralische Substanz aus einer amorphen Calciumphosphat-Phase und kristallinem, z.T. carbonathaltigem und durch Fehlordnungen gekennzeichnetem Apatit, vorzugsweise Hydroxylapatit. Apatit besitzt eine große innere Oberfläche, an der Ionenaustauschvorgänge möglich sind, wie z.B. der Austausch von Hydroxyl-Ionen gegen Fluorid oder CO₃²⁻-Ionen unter Bildung von Fluorapatit bzw. Carbonatapatit.

Die Steuerung der Stammzellen, die Apatit und sein wichtigstes Speicherprotein, das Kollagen, synthetisieren und dabei Calcium und Phosphor verbrauchen, erfolgt insbesondere durch das den Calciumhaushalt regulierende PTH, wobei die Stammmzellen zu Osteoblasten entwickelt werden.

Die für den Knochenabbau zuständigen Osteoklasten sind Zellverbände mit hoher Phagozytoseleistung (Makrophagen), die aus den Knochenmarkstammzellen entstehen. Sie werden durch Granulozyteopoese gebildet, gehören dem retikulo-endothelialen System an; machen ca. 5 - 8 % der Leukozytenzahl aus und siedeln sich im Knochen an. Hydroxylapatit wird im Knochenstoffwechsel von den Osteoklasten 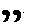 labilisiert", d.h. es erfolgt ein Abbau des Apatits bis zu einem Grundgerüst. Falls keine Störungen vorliegen, wird diese Grundsubstanz von Osteoblasten in die Interzellularräume des Knochens abgeschieden und zur weiteren Knochensynthese verwendet.

Unter dem Einfluß des PTH findet, wie bereits ausgeführt, der Transport von Calcium und Phosphat aus dem Knochen zu den entstehenden Osteoblasten statt. Umgekehrt werden nach der Synthese des Apatits Calcium und Phosphat in den Knochen transportiert. PTH, das die Aufgabe hat, möglichst viele Stammzellen zu Osteoblasten zu differenzieren, um möglichst viel Apatit zu produzieren, verbraucht dabei also Calcium und Phosphat. Das hat zur Folge, daß der Calcium- und Phosphat-Spiegel sinkt, wodurch wiederum ein Apatitverlust in den Knochen auftritt.

Demzufolge können folgende Schwachstellen des Knochenstoffwechsels auftreten:
a) ungenügende Differenzierung der Stammzellen durch PTH,
b) 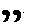 Labilisierung" des Hydroxylapatits durch Osteoklasten,
c) ungenügender Transport von Calcium und Phosphat zu den Osteoblasten.

Überraschend wurde nun gefunden, daß die Osteoblasten nur dann ausreichend mit Calcium und Phosphat versorgt werden, wenn der Knochen mit Hydroxylapatit geffüllt ist.

Gegenstand der Erfindung ist deshalb ein Kombinationspräparat aus PTH (bzw. einem PTH-Derviat) und Calcium- und/oder Phosphatverbindungen, das für die Therapie von Knochenstoffwechselstörvngen geeignet ist. Die Wirkstoffe des Kombinationspräparates können sowohl in getrennten Darreichungsformen als auch in einer Darreichungsform vorliegen.

Das Kombinationspräparat enthält neben PTH erfindungsgemäß bevorzugt eine Calciumphosphatkomplexverbindung. Ganz besonders bevorzugt enthält es eine Apatitverbindung der Formel Ca²⁺[Ca₃(PO₄)₂]₃²⁻, die vorzugsweise in Form eines Glukonates vorliegen kann. Das Kombinationspräparat kann aber auch als Calciumverbindung Calcium-Glukonat und/oder als Phosphatverbindung Glukose-1-Phosphat oder Kaliumhydrogenphosphat enthalten.

Das bevorzugte Verhältnis von Calcium zu Phosphat (bzw. Phosphor) beträgt im Kombinationspräparat von 11:5 bis 9:7, vorzugsweise 10:6.

Das Kombinationspräparat kann auch PTH nur in Kombination mit einer Calciumverbindung enthalten. Diese Präparate dienen insbesondere der Behandlung von Hypocalcämie. Präparate enthaltend PTH nur in Kombination mit einer Phosphatverbindung können insbesondere zur Behandlung von Hypophosphatämie eingesetzt werden.

In einer bevorzugten Variante enthält das Kombinationspräparat 0,5-20 µg PTH, 20-200 mg Calcium und/oder 10 - 100 mg Phosphor (bzw. 30 - 300 mg Phosphat). Diese Werte sind so zu verstehen, daß eine entsprechende Calcium- oder Phosphatverbindung in einer solchen Menge zu applizieren ist. daß 20 bis 200 mg Calcium bzw. 10 bis 100 mg Phosphor (bzw. 30 - 300 mg Phosphat) zugeführt werden.

Im Sinne der vorliegenden Erfindung sind unter dem Begriff 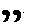 PTH" auch solche Peptide zu verstehen, die sich von dem natürlichen PTH-Peptid von 84 Aminosäuren durch Deletionen, Substitutionen oder Variationen einer oder mehrerer Aminosäuren ableiten. Insbesondere kommen C-terminal verkürzte Peptidfragmente in Frage, wie beispielsweise PTH(1-34), PTH(1-35), PTH(1-36) oder PTH(1-37). Derartige PTH-Derivate sind beispielsweise beschrieben in EP 0 497 915, WO 93/15109, EP 0 301 484 oder WO 90/10067. Erfindungsgemäß kann natürlich vorkommendes PTH, chemisch synthetisiertes oder gentechnisch hergestelltes PTH zur Anwendung kommen, insbesondere humanes PTH (hPTH). Auch PTH-Fragmente, die die gleiche Wirkung erzielen, können eingesetzt werden, wobei diese sowohl durch Spaltung des natürlich vorkommenden PTH als auch durch chemisch-synthetische oder gentechnische Verfahren hergestellt werden können (DE 3725319; Sömjen et al., Biochem. J. 272, 781-5 1990)).

Die Kombinationspräparate der vorliegenden Erfindung können PTH und die Calciumund/oder Phosphatverbindungen in einer verkaufsfertigen Verpackungseinheit nebeneinander konfektioniert enthalten (sogenannte Kombinationsverpackung). Die Arzneimittelpackungen können außerdem entweder eine geeignete Menge an PTH oder eine geeignete Menge einer Calcium-phosphatverbindung in Form eines Einzelpräparates enthalten, wobei die Einzelpräparate hinsichtlich der Menge der Inhaltsstoffe derart konfektioniert sind, daß sie im Sinne der Erfindung fiir die kombinierte Gabe mit dem jeweils anderen Präparat verabreicht werden können. In diesen Fällen werden vom Hersteller oder dem Arzneimittel-Importeur den Präparaten in der Regel ein in vielen Ländern gesetzlich vorgeschriebener Beipackzettel für Arzneimittel beigelegt, in dem Anweisungen oder Informationen über die kombinierte Gabe der Einzelpräparate enthalten sind. Dies trifft auch auf Arzneimittelpackungen zu, die eine geeignete Menge an PTH und eine geeignete Menge einer Calciumverbindung oder einer Phosphatverbindung enthalten bzw. einer Calciumverbindung und einer Phosphatverbindung.

Im Sinne der Erfindung kommen als Calcium-/Phosphat-präparate orale oder parenterale Darreichungsformen in Frage. Es kann sich gtundsätzlich um Einzelpräparate handein, die als Wirkstoff ein physiologisch verträgliches Calciumsalz und/oder eine Phosphatverbindung oder eine Calcium-phosphatkomplex-Verbindung enthalten, oder auch um Kombinationspräparate, die neben dem physiologisch verträglichen Präparaten weitere Wirkstoffe, wie z.B. Vitamine, Folsäure, Thiaminchluorid, Riboflavin, Pyridoxin, Ascorbinsäure, Nicotinarnid, etc. enthalten.

Es ist möglich, PTH und Calcium- und/oder Phosphatverbindungen in Form von getrennten pharmazeutischen Formulierungen (freie Kombination) gleichzeitig oder aber auch nacheinander zu applizieren. Diese freie Kombination, die in einer Verpackungseinheit zur Vertügung gestellt werden kann, hat den Vorteil der großen Flexibilität.

In der Regel wird die freie Kombination in Form einer einzigen Verpackungseinheit zur Verrügung gestellt, die mindestens zwei Behältnisse umfaßt, wobei das erste eine geeignete Darreichungsform für PTH ist (Lyophilisat, Injektions- oder Infusionslösung), und das zweite eine geeignete Darreichungsform tur das Calciumphosphat- oder Calcium- und/oder Phosphat-Präparat darstellt. Dadurch kann jedem Patienten individuell eine direkt zuzuordnende Menge an PTH und Calcium-/Phosphatverbindung zur Verfügung gestellt werden. Derartige Kombinationspräparate bieten darüber hinaus den Vorteil des größeren Therapieerfolges, da jeweils die optimal abgestimmte Menge der Einzelpräparate festgelegt ist, und deren Verwechslung mit sonst im Handel erhältlichen Einzelpräparaten, die in unterschiedlichen Dosierungen angeboten werden, weitgehend ausgeschlossen werden kann.

Die erfindungsgemäßen Kombinationspräparate minimieren ferner das Risiko einer versehentlich zu hohen Calcium- und/oder Phosphatgabe, die möglicherweise erfolgen kann, wenn herkömmliche Calcium- oder Phosphatpräparate aus separaten Arzneimittelpackungen zusammen mit PTH appliziert werden. Durch die erfindungsgemäßen Kombinationationspräparate wird eine sichere Therapie und einfache Handhabung sichergestellt. Im vorliegenden Fall ist es auch möglich, einen Wirkstoff als Injektionslösung und den anderen Wirkstoff als Darreichungsform zur oralen Verabreichung einzusetzen.

Für den Fall, daß PTH als Lyophilisat zur Verfügung gestellt wird, enthalten die Arzneimittelpackungen (Kombinationspackungen) die entsprechende Menge an PTH in Glasampullen oder in Karpulen. Das Calcium-/Phosphat-Präparat kann in fester Form (Tablette, Pulver, Granulat, Lyophilisat, etc.) oder auch in flüssiger Form in getrennten Behältnissen vorliegen. Ferner enthält die Kombinationspackung eine Rekonstitutionslösung, um entweder das PTH-Lyophilisat allein oder auch zusammen mit dem festen Calcium-/Phosphat-Präparat aufzulösen. Liegt das Calcium/Phosphat-Präparat als gebrauchsfertige Lösung vor, kann die Lösung zusammen mit der PTH-Lösung gemischt werden, falls die gemeinsame Applikation erfolgen soll. Grundsätzlich kann das Calcium-/Phosphat-Präparat auch als Konzentrat für den Zusatz zu herkömmlichen Irifusionslösungen zur Verfügung gestellt werden, wodurch eine langsamere Applikation über mehrere Stunden hinweg erfolgen kann.

Eine weitere Möglichkeit im Sinne der Erfindung besteht darin, jeweils Einzelpräparäte von PTH und Calcium-und/oder Phosphatverbindungen als unabhängige Arzneimittel zur Vertügung zu stellen, wobei die Einzelpräparate derart konfektioniert sind, daß sie die erforderlichen Mengen an Einzelsubstanzen für die erfindungsgemäße Kombination von PTH und Calcium und/oder Phosphat enthalten. Derartige Einzelpräparate können ferner entsprechende schriftliche Informationshinweise, beispielsweise in Form von Beipackzetteln oder Verpackungsaufdrucken, enthalten, die auf die kombinierte Gabe zusammen mit dem jeweils anderen Einzelpräparate in der erforderlichen Menge hinweisen. Derartige Informationen sind auch relevant im Zusammenhang mit der arzneimittelrechtlichen Genehmigung für das Inverkehrbringen derartiger Präparate bzw. dienen als Fachinformation im Arzneimittelmarkt.

Bei der Anwendung der Kombinationspräparate ist es auch möglich, PTH und Calciumund/oder Phosphatverbindungen in einer sogenannten fixen Kombination, d.h., in einer einzigen pharmazeutischen Formulierung zu verabreichen, in der beide Verbindungen enthalten sind. Dies kann z.B. eine Injektionslösung- bzw. Infusionslösung oder deren Lyophilisat sein, die beispielsweise in Ampullen abgefüllt sind. Die fixe Kombination der jeweiligen Wirkstoffe in Form eines Lyophilisates hat den Vorteil der einfachen und sicheren Handhabung. Das Lyophilisat wird in der Ampulle durch Zugabe pharmazeutisch üblicher Injektionsmedien gelöst und intravenös appliziert.

Die Herstellung der pharmazeutischen Darreichungsformen erfolgt nach üblichen, in der galenischen Technik bekannten Verfahren mit pharmazeutisch üblichen Hilfsstoffen.

Bei der Durchführung der Kombinationstherapie mit dem erfindungsgemäßen Kombinationspräparat muß mittels verschiedener diagnostischer Parameter der Füllzustand des Knochens mit Apatit und mit Kollagen festgestellt werden. Als besonders relevant und aussagekräftig haben sich hierbei herausgestellt:
a) die Bestimmung des Produktes Calcium x Phosphat im Serum,
b) die Bestimmung des Matrixproteins Osteocalcin im Serum,
c) die Bestimmung des Kollagens über seine Marker.

Die Zielwerte, die in der Regel auf eine gute Einstellung des Knochenstoffwechsels

hinweisen, können Tabelle 1 entnommen werden:

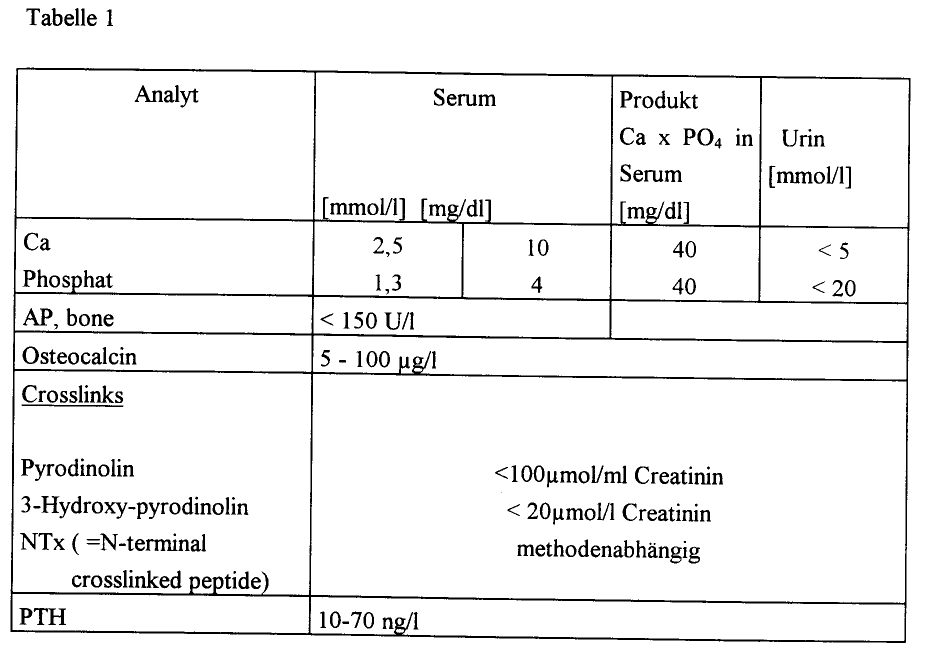

Bei der Durchführung der Therapie sind also deshalb im wesentlichen die folgenden diagnostischen Parameter zu kontrollieren:
a) Ca x PO₄ im Serum (in mg/dl), sowie Ca und Phosphat im Urin;
b) der Füllungsstand des Apatits im Kollagen über den Nachweis von bone ALP, Pyridinoline ( 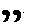 Crosslinks"), 3-Hydroxyprolin und NTx.
c) die Konzentration des Osteocalcins, das auch Apatit speichern und transportieren kann.

Das Produkt Calcium x Phosphat stellt für die diagnostischen Untersuchungen die wichtigste Größe dar und wird im Serum bestimmt. Es wurde gefunden, daß der Knochen ausreichend mit Ca und PO₄ gefüllt ist, wenn das Produkt Ca x PO₄ 30-50 mg/dl beträgt, wobei der Zielwert 40 mg/dl sein sollte. Als Anzeichen einer Organverkalkung ist ein Wert > 60 mg/dl Ca/PO₄ anzusehen. Bei Werten < 25 mg/dl drohen Hypocalcämie bzw. Hypophosphatämie.

Die Normalwerte für Calcium liegen im Serum bei 2,2 - 2,6 mmol/l, das entspricht 8,6 - 10,2 mg/dl (Calcium). Die Normalwerte für Phosphat liegen bei 1,0 - 1,5 mmo1/1, das entspricht 2,7 - 4,5 mg/dl, (Phosphat). Daraus resultiert ein Produkt tur Ca x PO₄, das bei Werten zwischen 253 mg/dl (8,6 x 2,7) und 46 mg/dl (10,2 x 4,5), als akzeptierbar angesehen werden kann.

Betrachtet man die Zusammensetzung des im Knochen vorkommenden Hydroxylapatits, so ist bei einem Produkt Ca x PO₄ von ca. 40 mg/dl eine optimale Beladungskapazität gegeben (vgl. Tabelle 2).

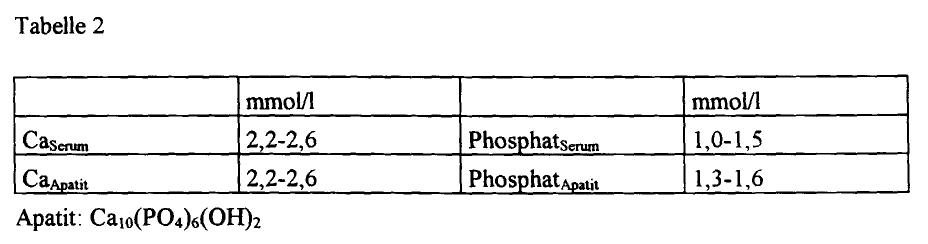

Der Nachweis des Kollagens, das das wichtigste Speicherprotein des Apatits ist, erfolgt indirekt über Marker des Knochenaufbaus im Serum, wie z.B. bone ALP und terminale Propeptide, die bei der Bildung von Kollagenfibrillen abgespalten werden, sowie über Marker des Knochenabbaus im Urin, wie Pyrodinoline (Crosslinks), 3-Hydroxy-Prolin oder NTx (N-terminal crosslinked peptide).

Auch das Matrixprotein Osteocalcin, dessen Glutaminsäurereste es befähigen, an die im Knochen eingelagerten Kristalle des Calcium-Minerals Hydroxyapatit zu binden, kommt im Serum in einer Konzentration von ca. 5 - 100 µg/l vor (Normalwert; methodenabhängig).

Bei der Durchführung der Kombinationstherapie kann mit dem erfindungsgemäßen Kombinationspräparat somit aufeinfache Art und Weise über die wöchentliche maximale Dosierung entschieden werden.

Die vorliegende Erfindung betrifft daher pharmazeutische Kombinationspräparate umfassend Parathyroidhormon (PTH) und Calcium- und/oder Phosphatverbindungen, wobei die Verbindungen in getrennten Darreichungsformen oder in einer einheitlichen Darreichungsform vorliegen können. Insbesondere kommen folgende Ausführungsformen in Frage: Kombinationspräparate, die PTH und eine Calcium-phosphatkomplexverbindung enthalten. Kombinationspräparate die als Calciumphosphatkomplexverbindung eine Apatitverbindung der Formel Ca²⁻[Ca₃(PO₄)₂]₃²⁻ enthalten, vorzugsweise in Form eines Glukonates. Kombinationspräparate, die PTH, eine Calcium- und ein Phosphatverbindung enthalten. Kombinationspräparate, die als Phosphatverbindung Glukose-1-Phosphat oder Kaliumhydrogenphosphat enthalten. Kombinätionspräparate, wobei das Verhältnis Calcium zu Phosphat zwischen 11:5 bis 9:7 liegt, und vorzugsweise 10:6 beträgt. Kombinationspräparate, die 0,5 - 20 µg PTH, 20 - 200 mg Calcium- und/oder 10 - 100 mg Phosphor (bzw. 30 - 300 mg Phosphat) enthalten. Pharmazeutische Verpackungseinheiten umfassend 0,5-20 µg PTH und 20-200 mg Calcium und/oder 10-100 mg Phosphor (bzw. 30 - 300 mg Phosphat) in getrennten Darreichungsformen als Injektions- bzw. Infusionslösungen oder als Lyophilisate oder in einer einheitlichen Darreichungsform.

Die vorliegende Erfindung betrifft ferner Verfahren zur Herstellung von pharmazeutischen Kombinationspräparaten wie oben angegeben, wobei man PTH, Calcium- und/oder Phosphatverbindungen mit pharmazeutisch üblichen Träger- oder Hilfsstoffen formuliert und in einer einheitlichen oder getrennten Darreichungsform zur Verfügung stellt. Die Erfindung bezieht sich ferner auf die Verwendung von PTH und Calciumund/oder Phosphatverbindungen zur Herstellung von Kombinationspräparaten zur Behandlung von Knochenstoffwechselstörungen.

Die Erfindung betrifft ferner Verfahren zur Bestimmung des Füllstandes des Knochens mit Hydroxylapatit in einer Probe aus Körperflüssigkeiten, wobei das Produkt Calcium x Phosphat sowie die Konzentrationen von Osteocalcin und von Kollagen bestimmt werden, insbesondere zur Bestimmung von Knochenstoffwechselstörungen.

Die Erfindung wird anhand des folgenden Beispiels näher erläutert.

### Beispiel:

Patienten mit manifesten Knochenstoffwechselstörungen, deren Osteocalcin-Wert unterhalb von 8 µl/l liegt und deren Calcium x Phosphat-Wert unterhalb von 20 liegt, werden dreimal pro Woche 10 mit µg hPTH behandelt. Außerdem erhalten die Patienten dreimal pro Woche 100 mg Calcium und 60 mg Phosphor, vorzugsweise in Form einer Ca[Ca₃(PO₄)₂]₃-Glukonat-Lösung, die infundiert wird. Diese Behandlung wird solange fortgeführt, bis der Osteocalcin-Wert und der Calcium x Phosphat-Wert im Normalbereich liegen (Dauer der Behandlung ca. fünf Wochen).

## Patentansprüche

1. Pharmazeutisches Kombinationspräparat umfassend Parathyroidhormon (PTH) und Calcium- und/oder Phosphatverbindungen, wobei die Verbindungen in getrennten Darreichungsformen oder in einer einheitlichen Darreichungsform vorliegen können.

2. Kombinationspräparat nach Anspruch 1, dadurch gekennzeichnet, daß es PTH und eine Calcium-phosphatkomplexverbindung enthält.

3. Kombinationspräparat nach Anspruch 2, dadurch gekennzeichnet, daß die Calciumphosphatkomplexverbindung eine Apatitverbindung der Formel Ca²⁺[Ca₃(PO₄)₂])²⁻ ist, vorzugsweise in Form eines Glukonates.

4. Kombinationspräparat nach Anspruch 1, dadurch gekennzeichnet, daß es PTH, eine Calcium- und ein Phosphatverbindung enthält.

5. Kombinationspräparat nach einem der Ansprüche 1 bis 4, dadurch gekennzeichnet, daß das Verhältnis Calcium zu Phosphat zwischen 11:5 bis 9:7, vorzugsweise 10:6 beträgt.

6. Kombinationspräparat nach einem der Ansprüche 1 bis 5, dadurch gekennzeichnet, daß es 0,5 - 20 µg PTH, 20 - 200 mg Calcium- und/oder 10 - 100 mg Phosphor enthält.

7. Pharmazeutische Verpackungseinheit umfassend 0,5-20 µg PTH und 20-200 mg Calcium und/oder 10-100 mg Phosphor in getrennten Darreichungsformen als Injektions- bzw. Infusionslösungen oder als Lyophilisate.

8. Verfahren zur Bestimmung des Füllstandes des Knochens mit Hydroxylapatit in einer Probe aus Körperflüssigkeiten, wobei das Produkt Calcium x Phosphat sowie die Konzentrationen von Osteocalcin und von Kollagen bestimmt werden.
